# EUROPEAN PATENT APPLICATION

(11) **EP 4 491 197 A1**
(43) Date of publication of application: **15.01.2025**
(21) Application number: 23766906.4
(22) Date of filing: 08.03.2023
(51) Int. Cl.: A61K 47/32, A61K 9/20, A61K 47/38

(54) **PHARMACEUTICAL COMPOSITION, PHARMACEUTICAL TABLET, AND METHOD FOR MANUFACTURING SAME**

(30) Priority: 10.03.2022 JP 2022037229
(71) Applicant: Mitsubishi Chemical Corporation, Tokyo 100-8251 (JP)
(72) Inventor: KOBAYASHI Ayaka, Tokyo 100-8251 (JP); YOSHIMURA Nobuyoshi, Tokyo 100-8251 (JP); MORIOKA Toshifumi, Tokyo 100-8251 (JP)
(74) Representative: Vossius & Partner Patentanwälte Rechtsanwälte mbB
(86) International application number: PCT/JP2023/008939
(87) International publication number: WO 2023/171730

(57) **Abstract**

The present invention provides a pharmaceutical composition, a pharmaceutical tablet, and a method for producing the same, which provide high tablet hardness and excellent sustained release. A pharmaceutical composition according to the present invention contains: a polyvinyl alcohol-based resin; an active pharmaceutical ingredient classified as a class III in BCS; and a cellulose, in which the polyvinyl alcohol-based resin has a ratio D50/D90 of D50 to D90 of 0.5 or less as measured using a laser diffraction particle size distribution measurement device, a content ratio of the polyvinyl alcohol-based resin to the cellulose is polyvinyl alcohol-based resin: cellulose = 80:20 to 99.9:0.1 in mass ratio, or the polyvinyl alcohol-based resin has a content of 40 mass% or more.

## Description

### TECHNICAL FIELD

The present invention relates to a pharmaceutical composition containing a polyvinyl alcohol-based resin, a pharmaceutical tablet, and a method for producing a pharmaceutical tablet.

### BACKGROUND ART

Currently, oral preparations such as tablets, capsules, granules, and powders are widely used as pharmaceutical dosage forms. The tablet, which is one of them, is generally produced by tablet forming. In a method of tablet forming, a granule obtained by granulating a powder mixture obtained by mixing various additive ingredients with an effective ingredient as a pharmaceutical (active pharmaceutical ingredient) is charged into a die, or the powder mixture is charged directly into a die, and formed into a desired size and shape by being compressed with a punch, so as to obtain a pharmaceutical tablet. The formed tablet may be coated as required.

A sustained-release tablet having a release behavior controlled to release the active pharmaceutical ingredient over a long period of time can maintain a constant concentration of the active pharmaceutical ingredient over a long period of time and avoid side effects when the number of administration is reduced. Examples of a method for controlling the release behavior of the active pharmaceutical ingredient include a method of coating a tablet with a resin or the like, and a method of dispersing an active pharmaceutical ingredient in a matrix base material such as a resin.

A polyvinyl alcohol (PVA)-based resin is a water-soluble polymer, and the use thereof as a matrix base material for dispersing the active pharmaceutical ingredient and allowing sustained release of the elution of the active pharmaceutical ingredient is being studied.

For example, Patent Literature 1 discloses a sustained-release pharmaceutical tablet that contains a polyvinyl alcohol-based resin having a degree of saponification of 65 mol% to 85 mol% and allows for elution control of an active pharmaceutical ingredient for a long period of time.

Patent Literature 2 discloses a co-mixture for a pharmaceutical preparation containing micronized polyvinyl alcohol (PVA) and a micronized microcrystalline cellulose (MCC) in combination with a micronized hydroxypropyl methyl cellulose (HPMC).

### CITATION LIST

### PATENT LITERATURE

Patent Literature 1: JP2020-152658A
Patent Literature 2: JP2020-510626A

### SUMMARY OF INVENTION

### TECHNICAL PROBLEM

However, the PVA-based resin has poor compression formability, and the pharmaceutical tablet containing a PVA-based resin disclosed in Patent Literature 1 has insufficient tablet hardness. The co-mixture disclosed in Patent Literature 2 enables sustained release by using HPMC in combination, but HPMC is difficult to handle because of a high viscosity thereof. Further, HPMC is known to have a poor fluidity during transfer because of its high charge rate.

In addition, when a content of the PVA-based resin in the pharmaceutical tablet is increased, it is possible to delay the release of the active pharmaceutical ingredient. However, the tablet hardness deteriorates when the content of the PVA-based resin is increased. Therefore, there has been a need for a pharmaceutical tablet that has both sustained release and tablet hardness.

From the viewpoint of production time and cost, it is preferable that a fewer ingredient is mixed.

Therefore, an object of the present invention is to provide a pharmaceutical tablet composition, a pharmaceutical tablet, and a method for producing the same, which provide high tablet hardness and excellent sustained release.

### SOLUTION TO PROBLEM

As a result of extensive research under these circumstances, the inventors of the present invention have found that when PVA having a non-uniform particle diameter is used and combined with a specific active pharmaceutical ingredient, even when a content of PVA is increased, tablet hardness does not deteriorate and both tablet hardness and sustained release can be achieved. Thus, the present invention has been completed.

That is, the gist of the present invention relates to the following <1> to <9>.
<1> A pharmaceutical composition containing:
   a polyvinyl alcohol-based resin;
   an active pharmaceutical ingredient classified as a class III in BCS; and
   a cellulose, in which
   a content ratio of the polyvinyl alcohol-based resin to the cellulose is polyvinyl alcohol-based resin: cellulose = 80:20 to 99.9:0.1 in mass ratio, and
   the polyvinyl alcohol-based resin has a ratio D50/D90 of D50 to D90 of 0.5 or less, as measured using a laser diffraction particle size distribution measurement device.
<2> A pharmaceutical composition containing:
   a polyvinyl alcohol-based resin;
   an active pharmaceutical ingredient classified as a class III in BCS; and
   a cellulose, in which
   the polyvinyl alcohol-based resin has a content of 40 mass% or more, and
   the polyvinyl alcohol-based resin has a ratio D50/D90 of D50 to D90 of 0.5 or less, as measured using a laser diffraction particle size distribution measurement device.
<3> The pharmaceutical composition according to the above <1> or <2>, in which the polyvinyl alcohol-based resin has a ratio D10/D90 of D10 to D90 of 0.25 or less, as measured using the laser diffraction particle size distribution measurement device.
<4>The pharmaceutical composition according to any one of the above <1> to <3>, in which the active pharmaceutical ingredient has a content of 20 mass% or more.
<5>The pharmaceutical composition according to any one of the above <1> to <4>, in which a content ratio of the polyvinyl alcohol-based resin to the active pharmaceutical ingredient is polyvinyl alcohol-based resin: active pharmaceutical ingredient = 10:90 to 90:10 in mass ratio.
<6> The pharmaceutical composition according to any one of the above <1> to <5>, in which the polyvinyl alcohol-based resin has an average degree of polymerization of 200 to 4,000.
<7> The pharmaceutical composition according to any one of the above <1> to <6>, in which the polyvinyl alcohol-based resin has an average degree of saponification of 70 mol% to 100 mol%.
<8> A pharmaceutical tablet containing:
   the pharmaceutical composition according to any one of the above <1> to <7>.
<9> A method for producing a pharmaceutical tablet, including:
   tableting the pharmaceutical composition according to any one of the above <1> to <7> into a tablet.

### ADVANTAGEOUS EFFECTS OF INVENTION

The pharmaceutical tablet containing the pharmaceutical composition according to the present invention has excellent tablet hardness and is thus difficult to break during forming or transportation, and further, has good sustained release and thus has good elution control during administration.

### DESCRIPTION OF EMBODIMENTS

The description of the constituent elements described below is an example of the embodiment of the present invention, and the present invention is not limited to these contents.

In the present description, D50/D90 and D10/D90 are mainly used to indicate the range of the particle diameter. In the present invention, "D10" means the value of the particle diameter at which the cumulative value is 10 vol% as a result of measurement using a laser analysis particle size distribution meter (dispersive pressure: 4 bar), "D50" means the value of the particle diameter at which the cumulative value is 50 vol% as a result of measurement using a laser analysis particle size distribution meter (dispersive pressure: 4 bar), and "D90" means the value of the particle diameter at which the cumulative value is 90 vol% as a result of measurement using a laser analysis particle size distribution meter (dispersive pressure: 4 bar).

In addition, in the present description, percent or part by mass has the same meaning as percent or part by weight.

A pharmaceutical composition according to the present invention contains a polyvinyl alcohol-based resin, an active pharmaceutical ingredient classified as a class III in BCS, and a cellulose.

A pharmaceutical composition according to a first embodiment is a pharmaceutical composition in which a content ratio of the polyvinyl alcohol-based resin to the cellulose is polyvinyl alcohol-based resin: cellulose = 80:20 to 99.9:0.1 in mass ratio, and the polyvinyl alcohol-based resin has a ratio D50/D90 of D50 to D90 of 0.5 or less, as measured using a laser diffraction particle size distribution measurement device.

A pharmaceutical composition according to a second embodiment is a pharmaceutical composition in which the polyvinyl alcohol-based resin has a content of 40 mass% or more, and the polyvinyl alcohol-based resin has a ratio D50/D90 of D50 to D90 of 0.5 or less, as measured using a laser diffraction particle size distribution measurement device.

### [Polyvinyl Alcohol-based Resin]

The polyvinyl alcohol (PVA)-based resin for use in the present invention is a resin mainly containing a vinyl alcohol structural unit, which is obtained by saponifying a polyvinyl ester-based resin obtained by polymerizing a vinyl ester-based monomer, and includes a vinyl alcohol structural unit equivalent to a degree of saponification and a vinyl ester structural unit in an unsaponified moiety.

Within in a range not impeding the effects of the present invention, it may be a modified polyvinyl alcohol-based resin obtained by saponifying a polyvinyl ester-based resin obtained by polymerizing a monomer copolymerizable with the vinyl ester-based monomer (copolymerizable monomer). The modification may be post-modification.

Examples of the vinyl ester-based monomer include vinyl formate, vinyl acetate, vinyl propionate, vinyl valerate, vinyl butyrate, vinyl isobutyrate, vinyl pivalate, vinyl caprate, vinyl laurate, vinyl stearate, vinyl benzoate, and vinyl versatate. Vinyl acetate is practically suitable.

Examples of the copolymerizable monomer include: olefins such as ethylene, propylene, isobutylene, α-octene, α-dodecene, and α-octadecene; hydroxy group-containing α-olefins such as 3-buten-1-ol, 4-penten-1-ol, 5-hexen-1-ol, and 3,4-dihydroxy-1-butene, and derivatives such as an acylated product thereof; unsaturated acids such as acrylic acid, methacrylic acid, crotonic acid, maleic acid, maleic anhydride, itaconic acid, and undecylenic acid, and salts thereof; nitriles such as acrylonitrile and methacrylonitrile; amides such as diacetone acrylamide, acrylamide, and methacrylamide; olefin sulfonic acids such as ethylene sulfonic acid, allyl sulfonic acid, and methallyl sulfonic acid, and salts thereof; vinyl compounds such as alkyl vinyl ethers, dimethylallyl vinyl ketone, N-vinyl pyrrolidone, vinyl chloride, vinyl ethylene carbonate, 2,2-dialkyl-4-vinyl-1,3-dioxolane, and glycerin monoallyl ether; substituted vinyl acetates such as isopropenyl acetate and 1-methoxyvinyl acetate; and hydroxymethylvinylidene diacetates such as vinylidene chloride, 1,4-diacetoxy-2-butene, 1,4-dihydroxy-2-butene, vinylene carbonate, 3,4-diacetoxy-1-butene, vinyl ethylene carbonate, glycerin monoallyl ether, 1,3-diacetoxy-2-methylenepropane, 1,3-dipropionyloxy-2-methylenepropane, and 1,3-dibutyronyloxy-2-methylenepropane. Such copolymerizable monomers may be used alone or in combination of two or more thereof.

In the present embodiment, it is preferable to use unmodified PVA as the PVA-based resin.

The unmodified PVA is obtained by subjecting a vinyl ester-based monomer to polymerization and saponification.

The unmodified PVA is a vinyl alcohol polymer having a vinyl alcohol structural unit equivalent to a degree of saponification and a vinyl ester structural unit in an unsaponified moiety.

As the PVA-based resin, among various PVA-based resins, one type can be used alone, or two or more types can be mixed and used in combination. When used in combination, PVA-based resins having different degrees of saponification and average degrees of polymerization, or modified PVA-based resins copolymerized with copolymerizable monomers can be used in combination.

The polymerization of the vinyl ester-based monomer (and the copolymerizable monomer contained if necessary) can be performed by known methods (such as bulk polymerization, solution polymerization, suspension polymerization, dispersion polymerization, or emulsion polymerization), and known polymerization catalysts and solvents used during polymerization can also be used. The solvent is preferably an alcohol, and more preferably a lower alcohol having 1 to 3 carbon atoms.

The saponification of the obtained vinyl ester-based polymer can be performed also by known methods. Industrially, the saponification can be performed using an alkali catalyst or an acid catalyst in a state where the polymer is dissolved in an alcohol or a water/alcohol solvent.

As the alkali catalyst, for example, hydroxides and alcoholates of alkali metals such as potassium hydroxide, sodium hydroxide, sodium methylate, sodium ethylate, potassium methylate, and lithium methylate can be used.

The saponification is suitably performed by a transesterification reaction using an alkali catalyst in the presence of an anhydrous alcohol-based solvent in terms of a reaction rate or a reduction of impurities such as fatty acid salts.

The reaction temperature in the saponification reaction is preferably 20°C to 60°C. When the reaction temperature is too low, the reaction rate tends to decrease and the reaction efficiency tends to decrease, and when the reaction temperature is too high, the temperature may be equal to or higher than a boiling point of the reaction solvent, which tends to lower the safety in production. Note that, when the saponification is performed under a high pressure using a tower type continuous saponification tower with high pressure resistance, the saponification can be performed at a higher temperature, for example, 80°C to 150°C, and it is also possible to obtain a PVA-based resin having a high degree of saponification in a short period of time even with a small amount of the saponification catalyst.

After the saponification, the obtained PVA-based resin is preferably washed with a washing liquid.

Examples of such a washing liquid include alcohols such as methanol, ethanol, isopropyl alcohol, and butanol. Methanol is preferred from the viewpoint of washing efficiency and drying efficiency.

The washing may be performed continuously, and batch washing is generally used. A bath ratio (mass of washing liquid to mass of PVA-based resin) is preferably 1 to 30, and particularly preferably 2 to 20. When the bath ratio is too large, a large washing device is required, and the cost tends to increase, and when the bath ratio is too small, the washing performance tends to be poor, and the number of washing tends to increase.

The washing temperature is preferably 10°C to 80°C, and particularly preferably 20°C to 70°C. When the washing temperature is too high, the vaporization amount of the washing liquid increases, and reflux equipment tends to be required. When the washing temperature is too low, the washing efficiency tends to decrease. The washing time is preferably 5 minutes to 12 hours. When the washing time is too long, the production efficiency tends to decrease, and when the washing time is too short, the washing tends to be insufficient. Further, the number of washing is preferably 1 to 10 times, and particularly preferably 1 to 5 times. When the number of washing is too large, the productivity is poor and the cost tends to increase.

The washed PVA-based resin particles are washed with a lower alcohol such as methanol and then dried with hot air or the like in a continuous or batch method to obtain a PVA-based resin powder. The drying temperature is preferably 50°C to 150°C, particularly 60°C to 130°C, and especially preferably 70°C to 110°C. When the drying temperature is too high, the PVA-based resin particles tend to be thermally deteriorated, and when the drying temperature is too low, it tends to take a long period of time for drying. The drying time is preferably 1 to 48 hours, and particularly preferably 2 to 36 hours. When the drying time is too long, the PVA-based resin particles tend to be thermally deteriorated, and when the drying time is too short, the drying tends to be insufficient or high temperature drying tends to be required.

The content of the solvent contained in the PVA-based resin after drying is preferably 0 mass% or more and 10 mass% or less, more preferably 0.1 mass% or more and 5 mass% or less, and still more preferably 0.1 mass% or more and 1 mass% or less.

The PVA-based resin generally contains an alkali metal salt of acetic acid derived from an alkali catalyst used during the saponification. The content of such an alkali metal salt of acetic acid is preferably 0.001 mass% or more and 2 mass% or less, more preferably 0.005 mass% or more and 1 mass% or less, and still more preferably 0.01 mass% or more and 0.1 mass% or less, with respect to the PVA-based resin.

When the content of the alkali metal salt is too large, the stability of the active pharmaceutical ingredient tends to decrease.

Examples of a method of adjusting the content of the alkali metal salt include a method of adjusting the amount of the alkali catalyst used during the saponification, or a method of washing the PVA-based resin with alcohols such as ethanol and methanol in the washing step.

Examples of a method for quantifying the alkali metal salt of the PVA-based resin for use in the present embodiment include a method of dissolving, for example, a PVA-based resin powder in water, and determining the content of the alkali metal salt through neutralization titration with hydrochloric acid, using methyl orange as an indicator.

The PVA-based resin preferably has an average degree of saponification of 70 mol% to 100 mol% (measured according to JIS K 6726). When such an average degree of saponification is 70 mol% or more, the water solubility tends to be good.

The average degree of saponification is preferably 70 mol% or more, more preferably 78 mol% or more, still more preferably 85 mol% or more, and particularly preferably 86 mol% or more. In addition, the average degree of saponification is preferably 100 mol% or less, more preferably 98 mol% or less, still more preferably 95 mol% or less, and particularly preferably 90 mol% or less.

The PVA-based resin preferably has an average degree of polymerization of 200 to 4,000 (measured according to JIS K 6726). When such an average degree of polymerization is 200 or more, the pharmaceutical tablet tends to have improved tablet hardness or sustained release performance, and when the average degree of polymerization is 4,000 or less, the tablet formability tends to be improved or the production efficiency tends to be improved.

The average degree of polymerization is preferably 200 or more, more preferably 1,500 or more, still more preferably 2,200 or more, and particularly preferably 2,450 or more. In addition, the average degree of polymerization is preferably 4,000 or less, more preferably 3,500 or less, and still more preferably 3,000 or less.

The PVA-based resin preferably has a viscosity of a 4 mass% aqueous solution of 2 mPa·s to 70 mPa·s at 20°C. When the viscosity of the 4 mass% aqueous solution is 70 mPa·s or less, the compression formability is improved, the tablet formability is improved, or the production efficiency is improved. On the other hand, when the viscosity of the 4 mass% aqueous solution is 2 mPa·s or more, the tablet hardness or the sustained release of the pharmaceutical tablet tends to be improved.

The viscosity of the 4 mass% aqueous solution is preferably 2 mPa·s or more, more preferably 20 mPa·s or more, still more preferably 30 mPa·s or more, and particularly preferably 46.5 mPa·s or more. In addition, the viscosity of the 4 mass% aqueous solution is preferably 70 mPa·s or less, and more preferably 60 mPa·s or less.

Note that in the present invention, the viscosity of the 4 mass% aqueous solution at 20°C can be measured by the following method. 500 g of water is mixed with 25 g of the PVA-based resin, the mixture is stirred at 60°C to 100°C to dissolve, and then water is added thereto to prepare a 4 mass% aqueous solution. The falling ball viscometer is immersed in a constant temperature water bath at 20°C for 30 minutes or longer, the aqueous solution is placed in the falling ball viscometer, and the viscosity of the 4 mass% aqueous solution is measured using a falling ball viscometer method according to JIS K6726.

The PVA-based resin for use in the present invention preferably has a large variation in particle size distribution. When PVA-based resins having various particle diameters are used, the sustained release is easily exhibited. The PVA-based resin having a large variation in particle size distribution for use in the present invention is not particularly limited, and can be obtained by pulverizing a PVA-based resin. Examples of means for pulverizing the PVA-based resin include a roller mill, a bead mill, a ball mill, a jet mill, a hammer mill, a pin mill, grind pulverization, freeze pulverization, and collision pulverization. Among them, freeze pulverization and collision pulverization are preferred because of being less influenced by to heat.

The freeze pulverization is a method in which a PVA-based resin is frozen using, for example, liquid nitrogen and then pulverized. The collision pulverization is a method in which a PVA-based resin is pulverized by self-collision using high-speed swirling airflow, or a method in which a PVA-based resin is pulverized by collision with a pulverization unit that moves at a high speed.

The PVA-based resin for use in the present invention may be a mixture of PVA-based resins obtained by pulverization with different methods.

In the present invention, the particle diameter refers to a particle diameter measured using a laser diffraction particle size distribution measurement device at a dispersive pressure of 4 bar, and the particle size distribution is calculated based on the particle diameter measured by this method.

In the case of the same PVA-based resin, the particle diameter obtained varies depending on the measurement conditions, particularly the dispersive pressure, so that in the present invention, the particle diameter is set as a particle diameter when measured at a dispersive pressure of 4 bar using a laser diffraction particle size distribution measurement device. Generally, as the dispersive pressure during the particle diameter measurement increases, particles in a lump state are more dispersed, so that the particle diameter tends to be more uniform.

Note that, there are a wet method and a dry method for measuring the particle diameter distribution using a laser diffraction method, and a dry method is used in the present invention.

As the particle diameter of the PVA-based resin, D10 (the value of the particle diameter at which the cumulative value is 10 vol%) when measured using a laser diffraction particle size distribution measurement device at a dispersive pressure of 4 bar is preferably 5 µm or more and 150 µm or less, more preferably 10 µm or more and 80 µm or less, and still more preferably 25 µm or more and 60 µm or less. In addition, D50 (the value of the particle diameter at which the cumulative value is 50 vol%, median diameter) is preferably 30 µm or more and 500 µm or less, more preferably 40 µm or more and 400 µm or less, and still more preferably 75 µm or more and 300 µm or less. In addition, D90 (the value of the particle diameter at which the cumulative value is 90 vol%) is preferably 50 µm or more and 700 µm or less, more preferably 70 µm or more and 500 µm or less, and still more preferably 200 µm or more and 450 µm or less.

As the particle size distribution of the PVA-based resin, the smaller the ratio D50/D90 of D50 to D90, the greater the variation in particle size distribution. In the present invention, a PVA-based resin having D50/D90 of 0.5 or less is used. D50/D90 is more preferably 0.1 or more and 0.48 or less, and still more preferably 0.2 or more and 0.45 or less.

When D50/D90 is 0.5 or less, the sustained release tends to be easily exhibited, and when D50/D90 is 0.1 or more, the particle diameter is uniform and segregation tends to be less likely to occur.

In addition, as the particle size distribution of the PVA-based resin, the smaller the ratio D10/D90 of D10 to D90, the greater the variation in particle size distribution. In the present invention, a PVA-based resin having D10/D90 of 0.25 or less is preferably used. D10/D90 is more preferably 0.1 or more and 0.22 or less, and still more preferably 0.1 or more and 0.2 or less.

When D10/D90 is 0.25 or less, the sustained release tends to be easily exhibited, and when D10/D90 is 0.1 or more, the particle diameter is uniform and segregation tends to be less likely to occur.

It is preferable that the PVA-based resin according to the present invention has D50/D90 of 0.5 or less and D10/D90 of 0.25 or less.

The content of the PVA-based resin in the pharmaceutical composition is 40 mass% or more. When such a content is 40 mass% or more, the sustained release of the tablet tends to be easily obtained. The content of the PVA-based resin is more preferably 42 mass% or more, and still more preferably 45 mass% or more. In addition, the PVA-based resin is preferably 80 mass% or less, more preferably 75 mass% or less, and still more preferably 70 mass% or less in the pharmaceutical composition. When the content of the PVA-based resin is 80 mass% or less, the tablet hardness of the tablet tends to be improved. That is, the content of the PVA-based resin in the pharmaceutical composition is preferably in the range of 40 mass% or more and 80 mass% or less.

The above PVA-based resin can be suitably used as a matrix base material (PVA-based resin for matrix) of a sustained-release pharmaceutical tablet.

Since the PVA-based resin is a water-soluble resin, it is thought that by dispersing the PVA-based resin as a matrix base material in a pharmaceutical tablet, the elution of the active pharmaceutical ingredient dispersed in the matrix is prevented, and it is possible to exhibit the sustained release.

It is thought that when such a PVA-based resin has a large variation in particle size distribution, during compression for tableting, particles having a small particle diameter efficiently enter gaps between particles having a large particle diameter, thereby improving the overall filling rate and improving the tablet hardness.

In addition, it is thought that the sustained release is improved since it is possible to construct a pseudo matrix gel in which the matrix PVA forms hydrogen bonds more densely.

### [Cellulose]

The cellulose is contained as an excipient in the pharmaceutical composition. Since the cellulose has excellent compression formability, the tablet hardness can be improved by containing such a cellulose.

Examples of the cellulose include a microcrystalline cellulose, sodium carboxymethyl cellulose, ethyl hydroxyethyl cellulose, hydroxypropyl methyl cellulose, hydroxyethyl methyl cellulose, hydroxypropyl cellulose, hydroxyethyl cellulose, ethyl methyl cellulose, ethyl cellulose, and methyl cellulose. The cellulose may be used alone or in combination of two or more thereof. Among them, a microcrystalline cellulose (for example, "Avicel" (trade name) manufactured by KOYO MERCANTILE CO., LTD.) is preferred. Here, the microcrystalline cellulose is a cellulose obtained by removing amorphous regions from a general cellulose.

The cellulose is preferably used in a powder form, and an average particle diameter of the cellulose for use in the present invention is not particularly limited, and is preferably 250 µm or less, more preferably 10 µm or more and 230 µm or less, still more preferably 20 µm or more and 150 µm or less, and particularly preferably 50 µm or more and 120 µm or less.

When the average particle diameter of the cellulose is 10 µm or more, the fluidity of the powder tends to be better, and when the average particle diameter is 250 µm or less, the compression formability tends to be better.

As the content ratio of the cellulose in the pharmaceutical composition, PVA-based resin: cellulose is 80:20 to 99.9:0.1, more preferably 83:17 to 99:1, and still more preferably 85:15 to 98:2 in mass ratio.

In terms of PVA-based resin: cellulose (mass ratio), when the content ratio of the PVA-based resin is 80 or more, the sustained release of the tablet tends to be easily obtained, and when the content ratio is 99.9 or less, the tablet hardness of the tablet tends to be improved.

### [Active Pharmaceutical Ingredient (API)]

As the active pharmaceutical ingredient that can be applied to the pharmaceutical composition according to the present invention, an active pharmaceutical ingredient as a class III in BCS (biopharmaceutics classification system) can be used.

The BCS class is a method of classifying compounds that can be active ingredients of pharmaceuticals according to the solubility and the membrane permeability thereof, and organizing the bioabsorption properties of the compounds corresponding to respective classes. The class III in BCS is for classifying drugs having high solubility and low membrane permeability, and is classified according to a biowaiver-based method based on ICH-M9 in the biopharmaceutics classification system (BCS).

Examples of the active pharmaceutical ingredient that can be used in the present invention include antipyretic analgesic antiphlogistics, nutrient and tonic supplements, psychotropics, antidepressants, antianxiety drugs, hypnosedatives, anticonvulsants, CNS-acting drugs, brain metabolism improving agents, brain circulation improving agents, antiepileptic agents, sympathomimetic drugs, gastrointestinal drugs, acid suppressants, antiulcerogenic drugs, cough medicines, antiemetics, anapnoics, bronchodilators, allergy medicines, antihistamine agents, agents for dental and oral use, cardiants, agents for cardiac arrhythmia, diuretics, antihypertensive drugs, vasoconstrictors, coronary vasodilators, peripheral vasodilators, blood coagulation inhibitors, hyperlipidemias agents, cholagogues, antibiotics, chemotherapeutic agents, diabetes drugs, osteoporosis drugs, antirheumatics, skeletal muscle relaxants, antispasmodics, hormonal agents, alkaloid drugs, sulfa drugs, arthrifuges, and antineoplastics.

Specific examples of the active pharmaceutical ingredient classified as the class III in BCS include abacavir, acyclovir, atenolol, baclofen, benznidazole, chloramphenicol, cimetidine, dapagliflozin, empagliflozin, ergocalciferol, famotidine hydrochloride, fexinidazole, fosfomycin, gabapentin, potassium glucosamine sulfate, lamivudine, levothyroxine sodium, linagliptin, lisinopril, metformin hydrochloride, methotrexate sodium, methyldopa, mirabegron, olopatadine hydrochloride, pantoprazole sodium, pentosan polysulfate sodium, proguanil hydrochloride, rabeprazole sodium, ranitidine hydrochloride, ribavirin, saxagliptin, sitagliptin, sofosbuvir, tenofovir disoproxil, thiamine hydrochloride, valacyclovir, vibegron, zinc sulfate, neostigmine bromide, and rizatriptan benzoate. In particular, metformin hydrochloride is preferably used, but the present invention is not limited thereto.

In addition, an active pharmaceutical ingredient that requires sustained release (particularly an active pharmaceutical ingredient for oral administration that requires sustained release since the active pharmaceutical ingredient is easily water-soluble) and an active pharmaceutical ingredient that is generally recognized to have poor compression formability can also be used.

A solid tablet composition according to the present invention can improve the formability, hardness, and strength while ensuring the sustained release. Therefore, even when used in combination with an active pharmaceutical ingredient that is generally recognized to have poor compression formability, it is possible to provide a sustained-release oral tablet having desired hardness and strength, which is particularly useful.

The content of the active pharmaceutical ingredient (API) in the pharmaceutical composition is preferably 20 mass% or more, more preferably 30 mass% or more, still more preferably 35 mass% or more, and particularly preferably 40 mass% or more. In addition, the content of the active pharmaceutical ingredient in the pharmaceutical composition is preferably 60 mass% or less, more preferably 58 mass% or less, still more preferably 55 mass% or less, and particularly preferably 53 mass% or less. That is, the content of the active pharmaceutical ingredient in the pharmaceutical composition is preferably in the range of 20 mass% or more and 60 mass% or less. When such a content is 20 mass% or more, the active pharmaceutical effect is sufficiently obtained, and when the content is 60 mass% or less, the sustained release of the tablet tends to be easily obtained.

In addition, as the content ratio of the PVA-based resin to the active pharmaceutical ingredient (API) in the pharmaceutical composition, PVA-based resin: API is preferably 10:90 to 90: 10, more preferably 20:80 to 80:20, and still more preferably 30:70 to 70:30 in mass ratio.

When PVA-based resin: API (mass ratio) is within the above range, both the tablet hardness and the sustained release can be achieved.

### [Other Additives]

In addition, the solid tablet composition according to the present invention may contain various additives within a range not impeding the effects of the present invention. Examples of other additives include a binder, a lubricant, an excipient, a disintegrant, a pH adjuster, a fluidizer, a surfactant, a colorant, a sweetener, and a coating agent.

Among them, a binder is added to provide binding properties between particles during dry or wet granulation, and tablet production by direct tableting or a wet production method. Such a binder is preferably used to control the disintegrability of a solid tablet formulation.

As the binder, dextrin, gum arabic, gelatin, hydroxypropyl starch, hypromellose, pullulan, starch paste, a polyvinyl alcohol-based resin, or the like can be used.

Here, a polyvinyl alcohol-based resin used as a binder (PVA-based resin for binder) has a viscosity of a 4 mass% aqueous solution at 20°C of preferably 2 mPa·s or more and 30 mPa·s or less, more preferably 2 mPa·s or more and 20 mPa·s or less, and particularly preferably 2 mPa-s or more and 10 mPa-s or less.

In addition, the PVA-based resin for binder has an average degree of saponification of preferably 70 mol% or more and 99 mol% or less, and more preferably 80 mol% or more and 90 mol% or less.

Note that, as the polyvinyl alcohol-based resin that can be used as a binder, a saponified copolymer with a copolymerizable monomer (modified polyvinyl alcohol-based resin), as described in connection with the polyvinyl alcohol-based resin used for the matrix base material, may be used as long as the viscosity of the 4 mass% aqueous solution is within the above range.

The polyvinyl alcohol-based resin used as a binder is preferably 1 mass% or more and 10 mass% or less, and more preferably 1 mass% or more and 5 mass% or less in the pharmaceutical composition.

Note that, in the case of being used as the binder, the polyvinyl alcohol-based resin is preferably used in the form of an aqueous solution by dissolution in water.

The lubricant is contained to improve the fluidity, and is thus added to provide lubricity during compression and tablet release in a die, and to prevent the tablet from adhering to a punch surface or a wall of the die. Specifically, stearic acid, magnesium stearate, calcium stearate, talc, silicic anhydride, or the like can be used.

Examples of the excipient that can be used include sugar alcohols (such as mannitol, erythritol, xylitol, sorbitol, and maltitol), sugars (such as glucose, fructose, lactose, sucrose, trehalose, maltose, and oligosaccharide), calcium phosphates, starches, sodium phosphates, and gelatin. Among them, sugar alcohols, particularly mannitol, are preferably used.

As other additives, disintegrants (carmellose calcium, sodium carboxymethyl starch, croscarmellose sodium, crospovidone, cellulose or derivatives thereof, and starch or derivatives thereof); pH adjusters (such as citric acid and salts thereof, phosphoric acid and salts thereof, carbonic acid and salts thereof, tartaric acid and salts thereof, fumaric acid and salts thereof, acetic acid and salts thereof, amino acids and salts thereof, succinic acid and salts thereof, and lactic acid and salts thereof); fluidizers (such as light anhydrous silicic acid, hydrous silicon dioxide, titanium oxide, stearic acid, corn gel, and heavy anhydrous silicic acid); surfactants (such as phospholipids, glycerin fatty acid esters, polyoxyethylene fatty acid esters, sorbitan fatty acid esters, polyethylene glycol fatty acid esters, polyoxyethylene hydrogenated castor oil, polyoxyethylene alkyl ethers, sucrose fatty acid esters, sodium lauryl sulfate, polysorbates, sodium hydrogen phosphates, and potassium hydrogen phosphates); colorants (such as iron sesquioxide, yellow iron sesquioxide, food yellow 5, food yellow 4, aluminum chelate, titanium oxide, and talc); and sweeteners (such as saccharin, aspartame, acesulfame potassium, thaumatin, and sucralose) may be contained.

### [Pharmaceutical Tablet and Method for Producing Same]

A pharmaceutical tablet according to the present invention contains the pharmaceutical composition according to the present invention. The pharmaceutical tablet is not particularly limited, and is, for example, produced by mixing the above various ingredients, followed by tablet forming, either directly or after granulation.

In the present invention, a related tablet production method can be applied as long as it is a tablet forming method that enables homogeneous dispersion of the active pharmaceutical ingredient in the PVA-based resin for matrix.

Specifically, a compression tableting method such as a direct powder compression method (direct compression method), a semi-dry granule compression method, and a granule compression method, or a wet production method can be used.

The direct powder compression method is a method in which drugs and additives are directly mixed and compression-formed, without adding water. In a dry granule compression method, granulation (slugging) is performed to obtain lumps using a dry method, followed by tableting. The dry granule compression method provides better homogeneity than the direct powder compression method. The semi-dry granule compression method or a semi-direct compression method is a method in which granules containing only additives and no drug are granulated using a wet method, and a drug is added thereto, followed by tableting. By producing wet granules, the binding strength of the tablet can be increased and powdering of the surface can be prevented. The wet granule compression method is a method of tableting granules obtained by granulating drugs and additives using a wet method. The wet production method is a method in which a wet kneaded material is poured into a certain direction or punched out, and then dried to remove solvents such as water.

In the above production method, it is preferable that a granulated material containing a PVA-based resin for binder and an active pharmaceutical ingredient is first produced, and such a granulated material is mixed with a PVA-based resin for matrix and other ingredients such as a cellulose as necessary, followed by tablet forming.

In the granulation process, a mixture obtained by adding and mixing other additives in addition to the PVA-based resin for binder and the active pharmaceutical ingredient may be granulated.

Examples of the granulation method include: a wet method in which a binder solution is added to a powder and direct granulation is performed, or a wet mass that has been kneaded once is granulated; a dry method in which a powder is compression-formed in a dry state and then crushed and granulated; and a spray granulation method in which a powder is slurried with a large amount of water and then granulated by spray drying.

Note that, the solvent used in the binder solution is appropriately selected depending on the type of the compound used as the binder. When a PVA-based resin is used as the binder, water can be used as the solvent.

As a granulator, a dry granulator (that applies a strong pressure to a powder to form a lump in a dry state, and then crushes the lump to form a particulate having an appropriate particle diameter), a basket type extrusion granulator, a stirring granulator (that mixes raw material powders for several minutes with a stirring blade, then adds a binder solution dropwise to perform stirring granulation), a centrifugal rolling granulator, a fluidized bed granulator (that mixes powder raw materials by fluidization with air, then sprays a binder solution from a nozzle using convection or countercurrent flow to agglomerate the powder raw materials with spray droplets, and then performs drying to promote particle growth), a spray dryer (spray drying granulator), or the like can be used.

A wet granulation treatment method is, for example, a treatment in which each ingredient is kneaded with a solvent and then granulated. Examples of this treatment include a crushing granulation method, an extrusion granulation method, a stirring granulation method, and a rolling granulation method. In addition, in this treatment, alcohols such as ethanol and isopropanol, and water are used as the solvent.

The granulated material is then subjected to tablet forming. The PVA-based resin for matrix and the cellulose are preferably mixed with the granulated material during tableting, and preferably a lubricant is also added and mixed in order to improve the compression formability.

The tablet forming may be performed by using any of production methods generally used for compression forming of solid tablets in the pharmaceutical field, including methods using a rotary tableting machine and methods using a single-press tableting machine. For example, the pharmaceutical tablet can be produced by using a direct powder compression method in which various ingredients are tableted after being uniformly mixed, or a wet granule compression method or dry granule compression method in which various ingredients are granulated by wet granulation or dry granulation and then the obtained granules are tableted.

Among them, it is preferable to use a wet granule compression method from the viewpoint of improving the fluidity and the mixing uniformity.

The compression pressure (tableting pressure) in the compression forming is preferably about 1 kN or more, more preferably about 2 kN or more, and still more preferably about 4 kN or more. In addition, the compression pressure is preferably about 60 kN or less, more preferably about 50 kN or less, and still more preferably about 30 kN or less. When the compression pressure is within this range, there is less burden on a die and a punch during tableting, and it is easy to maintain a constant tableting pressure during tableting.

The pharmaceutical tablet may have any shape, including an ellipsoid, a column, a doughnut, and a sphere. The obtained tablet may be further coated with a film to form a coated tablet, if necessary.

The sustained-release pharmaceutical tablet produced as described above generally has hardness of preferably 40 N to 250 N, more preferably 50 N to 200 N, and still more preferably 60 N to 190 N, depending on the form and the size of the tablet. When the tablet hardness satisfy the above range, the properties required for a tablet can be satisfied.

In addition, the sustained release is possible regardless of the water solubility of the active pharmaceutical ingredient, depending on the type of the composition, particularly the degree of polymerization and the average degree of saponification of the polyvinyl alcohol-based resin, and the type and the content ratio of the active pharmaceutical ingredient.

The elution rate varies depending on the intended use. In the use of the present invention, the elution rate of the active pharmaceutical ingredient is preferably 45% or less, and more preferably 42% or less up to 1 hour after administration, and is preferably 75% or less, and more preferably 72% or less up to 3 hours after administration. The final elution rate is preferably 75% or more, more preferably 80% or more, and still more preferably 82% or more. The closer it is to 100%, the more preferable it is.

### Examples

Hereinafter, the present invention will be further described with reference to Examples and Comparative Examples, but the present invention is not limited to the following Examples.

### [Preparation of Polyvinyl Alcohol]

### (PVA1)

Into a 1000 mL eggplant flask, 100 g of a 10 wt% PVA aqueous solution of a PVA powder (degree of saponification: 88 mol%, degree of polymerization: 2,500) was charged, preliminary drying with liquid nitrogen was performed, then the degree of vacuum of a freeze dryer ("FDU-1200" manufactured by TOKYO RIKAKIKAI CO., LTD.) was adjusted to 20 Pa or less, and the freeze dryer was operated until the frost on the surface of the eggplant flask was thawed, to obtain a freeze-dried PVA dry powder.

The obtained PVA dry powder was pulverized using a freeze pulverizer ("Freezer Mill 6770" manufactured by SPEX SamplePrep LLC) under the conditions of cycles: 3, precool: 1 min, run time: 1 min, cool time: 1 min, rate: 10 CPS, to obtain a pulverized PVA1.

The particle diameter of the obtained PVA1 was measured using a laser diffraction particle size distribution measurement device (Mastersizer-3000 manufactured by Malvem) at a dispersive pressure of 4 bar and a measurement time of 1 second, and D10, D50, and D90 were determined.

The results are summarized in Table 1.

### (PVA2)

A PVA powder (degree of saponification: 88 mol%, degree of polymerization: 2,500) was subjected to collision pulverization using a fine pulverizer ("ACM-15H" manufactured by Hosokawa Micron Group) under the conditions of pulverizing rotation speed: 7,800 rpm, hammer shape: vertical grooves, number of hammers: 16, and classification rotation speed: 1,250 rpm, to obtain first PVA.

Second PVA was similarly obtained by collision pulverization while changing the conditions in the collision pulverization to pulverizing rotation speed: 7,800 rpm, hammer shape: vertical grooves, number of hammers: 16, and classification rotation speed: 3,000 rpm.

The first PVA and the second PVA were mixed at a mass ratio of 1:1, to obtain PVA2.

The particle size distribution of the obtained PVA2 was determined in the same manner as PVA1. The results are summarized in Table 1.

### (PVA3)

A PVA powder (degree of saponification: 88 mol%, degree of polymerization: 2,500) was not pulverized and was used as PVA3.

The particle size distribution of PVA3 was determined in the same manner as PVA1. The results are summarized in Table 1.

### (PVA4)

A PVA powder (degree of saponification: 88 mol%, degree of polymerization: 2,500) was subjected to collision pulverization using a fine pulverizer ("ACM-15H" manufactured by Hosokawa Micron Group) under the conditions of pulverizing rotation speed: 7,800 rpm, hammer shape: vertical grooves, number of hammers: 16, and classification rotation speed: 1,300 rpm, to obtain first PVA.

Second PVA was similarly obtained by collision pulverization while changing the conditions in the collision pulverization to pulverizing rotation speed: 7,800 rpm, hammer shape: vertical grooves, number of hammers: 16, and classification rotation speed: 3,000 rpm.

The first PVA and the second PVA were mixed at a mass ratio of 1:1, to obtain PVA4.

The particle size distribution of the obtained PVA4 was determined in the same manner as PVA1. The results are summarized in Table 1.

### [Table 1]

**Table 1**

| | D10 (µm) | D50 (µm) | D90 (µm) | D50/D90 | D10/D90 |
|---|---|---|---|---|---|
| PVA1 | 46 | 180 | 412 | 0.44 | 0.11 |
| PVA2 | 27 | 60 | 141 | 0.43 | 0.19 |
| PVA3 | 141 | 276 | 482 | 0.57 | 0.29 |
| PVA4 | 24 | 58 | 153 | 0.38 | 0.16 |

### [Preparation of Tablet]

### (Example 1)

A PVA aqueous solution (concentration: 20%) obtained by dissolving 1.1 parts by mass of a PVA-based resin (degree of saponification: 88 mol%, viscosity of 4 mass% aqueous solution: 3.4 mPa·s, D50/D90 = 0.575) as a binder in water was added to 47.7 parts by mass of metformin hydrochloride as an active pharmaceutical ingredient classified as a class III in BCS, and the mixture was granulated using a granulation device ("FM-VG-01" manufactured by Powrex Corp.).

To the obtained granulated material, 46.3 parts by mass of PVA1 as a matrix ingredient, 5 parts by mass of a microcrystalline cellulose (average particle diameter: 90 µm), and 1 part by mass of magnesium stearate were added, and this mixed composition was tableted using a rotary tableting machine ("HT-EX12SS-U" manufactured by HATA TEKKOSHO CO., LTD.) at a tableting pressure of 25 kN, to prepare a pharmaceutical tablet (diameter: 11 mm, mass: 500 mg).

The obtained pharmaceutical tablet was evaluated as follows. The results are shown in Table 2.

### (Example 2)

A pharmaceutical tablet was prepared in the same manner as in Example 1 except that PVA2 was used as the matrix ingredient.

The obtained pharmaceutical tablet was evaluated as follows. The results are shown in Table 2.

### (Example 3)

A pharmaceutical tablet was prepared in the same manner as in Example 2 except that PVA2 was 44.1 parts by mass and the microcrystalline cellulose was 7.2 parts by mass.

The obtained pharmaceutical tablet was evaluated as follows. The results are shown in Table 2.

### (Comparative Example 1)

A pharmaceutical tablet was prepared in the same manner as in Example 1 except that PVA3 was used as the matrix ingredient.

The obtained pharmaceutical tablet was evaluated as follows. The results are shown in Table 2.

### (Comparative Example 2)

A pharmaceutical tablet was prepared in the same manner as in Example 1 except that PVA4 was used as the matrix ingredient and ascorbic acid was used as the active pharmaceutical ingredient as class I in BCS.

The obtained pharmaceutical tablet was evaluated as follows. The results are shown in Table 2.

### (Comparative Example 3)

A pharmaceutical tablet was prepared in the same manner as in Example 1 except that PVA1 was 25.65 parts by mass and the microcrystalline cellulose was 25.65 parts by mass.

The obtained pharmaceutical tablet was evaluated as follows. The results are shown in Table 2.

### [Measurement and Evaluation Method]

### [Sustained Release]

An elution test for the tablet was performed based on a DISSOLUTION Test 1 in "Metformin Hydrochloride extended-Release Tablets" in USP41.

Using an elution tester ("NTR-6400ACT" manufactured by Toyama Sangyo Co., Ltd.), the tablet was subjected to an elution test in 900 mL of phosphoric acid buffer (pH: 6.8) whose temperature was adjusted to 37°C. The elution rate (%) of the active pharmaceutical ingredient (metformin hydrochloride or ascorbic acid) 1 hour, 3 hours, and 10 hours after the start of the elution test was measured using an ultraviolet absorption photometer ("PAC-743" manufactured by JASCO Corporation), and the sustained release was evaluated according to the following evaluation criteria A to C. When both the evaluation criteria A and B are satisfied, A was given.

A (good): the elution rate 1 hour after the start of the elution test is 20% or more and 45% or less, the elution rate after 3 hours is 50% or more and 75% or less, and the elution rate after 10 hours is 80% or more and 100% or less.

B (acceptable): the elution rate 1 hour after the start of the elution test is 15% or more and 45% or less, the elution rate after 3 hours is 45% or more and 75% or less, and the elution rate after 10 hours is 75% or more and 100% or less.

C (unacceptable): the elution rate 1 hour after the start of the elution test is less than 15%, or the elution rate after 3 hours is more than 80%.

### [Hardness Evaluation]

The tablet hardness after tableting was measured using a hardness meter PC-30 (manufactured by Okada Seiko Co., Ltd.). Those having tablet hardness of more than 100 N were evaluated as "A (good)", those having tablet hardness of 50 N or more and less than 100 N were evaluated as "B (acceptable)", and those having tablet hardness of less than 50 N evaluated as "C (unacceptable)".

### [Table 2]

**Table 2**

| | PVA | Active pharmaceutical ingredient | Elution rate (%) | | | Sustained release | Tablet hardness (N) | |
|---|---|---|---|---|---|---|---|---|
| | | | 1h | 3h | 10h | | | |
| Example 1 | PVA1 | Metformin | 40.6 | 64.3 | 85.2 | A | 182 | A |
| Example 2 | PVA2 | Metformin | 40.6 | 71.3 | 92.8 | A | 69 | B |
| Example 3 | PVA2 | Metformin | 36.9 | 60.4 | 75.7 | B | 56 | B |
| Comparative Example 1 | PVA3 | Metformin | 92.8 | 94.5 | 96.5 | C | 12 | C |
| Comparative Example 2 | PVA4 | Ascorbic acid | 35.1 | 69.1 | 77.4 | B | 10 | C |
| Comparative Example 3 | PVA1 | Metformin | 47.6 | 83.4 | 94.4 | C | 158 | A |

As seen from the results in Table 2, in the case of using PVA having a large particle size distribution as a matrix, when an active pharmaceutical ingredient classified as a class III in BCS is used, a tablet having excellent sustained release and tablet hardness can be obtained.

Although the present invention has been described in detail with reference to the specific embodiment, it will be apparent to those skilled in the art that various changes and modifications can be made without departing from the spirit and scope of the present invention. The present application is based on a Japanese Patent Application (No. 2022-037229) filed on March 10, 2022, contents of which are incorporated herein by reference.

### INDUSTRIAL APPLICABILITY

The pharmaceutical tablet containing the pharmaceutical composition according to the present invention has sustained release and sufficient hardness. Therefore, the pharmaceutical composition and the pharmaceutical tablet according to the present invention are useful because of being capable of being combined with various active pharmaceutical ingredients for which sustained-release tablets are desired to provide a desired sustained-release tablet.

## Claims

1. A pharmaceutical composition comprising:
a polyvinyl alcohol-based resin;
an active pharmaceutical ingredient classified as a class III in BCS; and
a cellulose, wherein
a content ratio of the polyvinyl alcohol-based resin to the cellulose is polyvinyl alcohol-based resin: cellulose = 80:20 to 99.9:0.1 in mass ratio, and
the polyvinyl alcohol-based resin has a ratio D50/D90 of D50 to D90 of 0.5 or less, as measured using a laser diffraction particle size distribution measurement device.

2. A pharmaceutical composition comprising:
a polyvinyl alcohol-based resin;
an active pharmaceutical ingredient classified as a class III in BCS; and
a cellulose, wherein
the polyvinyl alcohol-based resin has a content of 40 mass% or more, and
the polyvinyl alcohol-based resin has a ratio D50/D90 of D50 to D90 of 0.5 or less, as measured using a laser diffraction particle size distribution measurement device.

3. The pharmaceutical composition according to claim 1 or 2, wherein the polyvinyl alcohol-based resin has a ratio D10/D90 of D10 to D90 of 0.25 or less, as measured using the laser diffraction particle size distribution measurement device.

4. The pharmaceutical composition according to any one of claims 1 to 3, wherein the active pharmaceutical ingredient has a content of 20 mass% or more.

5. The pharmaceutical composition according to any one of claims 1 to 4, wherein a content ratio of the polyvinyl alcohol-based resin to the active pharmaceutical ingredient is polyvinyl alcohol-based resin: active pharmaceutical ingredient = 10:90 to 90:10 in mass ratio.

6. The pharmaceutical composition according to any one of claims 1 to 5, wherein the polyvinyl alcohol-based resin has an average degree of polymerization of 200 to 4,000.

7. The pharmaceutical composition according to any one of claims 1 to 6, wherein the polyvinyl alcohol-based resin has an average degree of saponification of 70 mol% to 100 mol%.

8. A pharmaceutical tablet comprising:
the pharmaceutical composition according to any one of claims 1 to 7.

9. A method for producing a pharmaceutical tablet, comprising:
tableting the pharmaceutical composition according to any one of claims 1 to 7 into a tablet.
